# EUROPÄISCHE PATENTSCHRIFT

(11) **EP 1 753 389 B1**
(45) Veröffentlichungstag und Bekanntmachung des Hinweises auf die Patenterteilung: **04.08.2010**
(21) Anmeldenummer: 05728242.8
(22) Anmeldetag: 30.03.2005
(51) Int. Cl.: A61Q 1/02, A61K 8/58, A61K 8/81, A61K 8/25, A61K 8/29, A61K 8/87

(54) **ZUBEREITUNG IN FORM EINER EMULSION MIT EINER FILMBILDENDEN POLYMERKOMBINATION**
PREPARATION PROVIDED IN THE FORM OF AN EMULSION HAVING A FILM-FORMING POLYMER COMBINATION
PREPARATION SOUS FORME D'EMULSION RENFERMANT UNE COMBINAISON POLYMERE FILMOGENE

(30) Priorität: 02.04.2004 EP 04008126; 08.06.2004 DE 102004027838
(43) Veröffentlichungstag der Anmeldung: 21.02.2007
(73) Patentinhaber: Schwan-STABILO Cosmetics GmbH & Co. KG, 90562 Heroldsberg (DE)
(72) Erfinder: BAUER, Barbara, 84508 Burgkirchen (DE)
(74) Vertreter: Leissler-Gerstl, Gabriele
(86) Internationale Anmeldenummer: PCT/EP2005/003329
(87) Internationale Veröffentlichungsnummer: WO 2005/094775

(56) Entgegenhaltungen:
- EP-A- 0 832 645
- EP-A- 0 987 016
- EP-A- 1 213 010
- EP-A- 1 541 126
- WO-A-03/094868
- US-B1- 6 491 931

## Beschreibung

Die Erfindung betrifft eine kosmetische Zubereitung in Form einer Emulsion, die zum Auftragen auf Haut und Schleimhaut, insbesondere im Bereich von Gesicht und Hals geeignet ist.

Kosmetische Produkte, die zur Verbesserung des Aussehens verwendet werden und auf Haut und/oder Schleimhaut, insbesondere im Bereich von Gesicht und Hals aufgetragen werden, sind in vielfältigen Ausführungsformen bekannt. In diese Zusammensetzungen werden üblicherweise Stoffe eingearbeitet, die der Haut vorteilhafte Eigenschaften verleihen sollen oder das Aussehen verbessern sollen. So ist es bekannt, in Hautpflegezusammensetzungen teilchenförmige Materialien einzuarbeiten, die feine Linien, Falten, Poren und andere unerwünschte Formen der Hautoberfläche kaschieren, indem sie Licht in geeigneter Weise reflektieren. Dies wird z.B. in "Quantification of the Soft-Focus Effect", Cosmetics and Toiletries, Band 111, Juli 1996, Seiten 57 bis 61 beschrieben unter Hinweis auf Substanzen wie TiO₂. Weiterhin sind aus US-A 4892726 Polymethylsilsesquioxanpulver zum Einarbeiten in Make-up- und Kosmetikzusammensetzungen bekannt, die der Haut nach dem Aufbringen ein glattes Aussehen und eine natürliche Farbe verleihen. Entsprechende Produkte sind unter der Bezeichnung "tospearl^{®}" erhältlich. US-A 5223559 beschreibt die Verwendung kleiner Teilchen mit einer Größe im Bereich von 0.5 bis 50 µm, um Hautdefekte zu kaschieren. Weiterhin ist aus EP-A 692 242 die Verwendung von hohlen Teilchen mit einer Größe im Bereich von 1 bis 250 µm zum selben Zweck bekannt. Auch andere Teilchen, wie Nylonteilchen wurden in kosmetischen Zusammensetzungen bereits angewendet.

Aus EP-A 1 156 772 sind topische Zusammensetzungen bekannt, die nicht für das Gesicht vorgesehen sind, sondern zum Auftragen auf andere Körperteile, insbesondere die Hände, die ein teilchenförmiges Material In Kombination mit speziellen Pigmenten und Mattierungsmitteln in einem dermatologisch annehmbaren topischen Träger enthalten.

WO 03/094868 A beschreibt Zubereitungen zum Auftragen auf die Haut oder Hautanhangsgebilde auf wässriger Basis, die eine Mischung aus mindestens zwei wässrigen Dispersionen filmbildender Polymere enthalten. Es werden "einphasige" Systeme mit Suspensionscharakter beschrieben, die keine emulgierte, ein flüchtiges Silikon enthaltende innere Phase enthalten. Nicht beschrieben wird die Verwendung von kugelförmigen feinen Pigmenten in Form von "light diffusing pigments" kurz "LDP".

EP 1 213 010 A2 beschreibt die Verwendung von filmbildenden wasserlöslichen Polymeren zur Erhöhung des Lichtschutzfaktors kosmetischer Zubereitungen. Die Schrift offenbart Zubereitungen, die auch Pigmente enthalten können, jedoch keine kugelförmigen Pigmenten in Form von LDP aufweisen. Aufgrund der besonderen Verwendung der Polymere als Mittel zur Erhöhung des Lichtschutzfaktors von kosmetischen Zubereitungen ist die Einsatzmenge der Polymere sehr gering, so dass nicht von einer, für die vorliegende Anwendung erforderlichen ausreichenden Filmbildung ausgegangen werden kann.

EP 1 541 124 A1 offenbart Zubereitungen zur Prophylaxe und Behandlung von sonnengereizter Haut, die auch Pigmente enthalten können, jedoch werden keine kugelförmigen feinen Pigmente in Form von LDP eingesetzt.

EP 0 987 016 A1 beschreibt Zusammensetzungen, die ein Spannung verleihendes Polymer und einen Polyester in Form eines dendritischen Polymers enthalten. Nicht eingesetzt werden Pigmente, insbesondere keine kugelförmigen Pigmente in Form von LDP.

Die bekannten kosmetischen Zusammensetzungen zum topischen Auftragen basieren alle auf wachs-, fett- und/oder ölhaltigen Grundstoffen, die sich als nachteilig herausgestellt haben. Wenn eine derartige Zusammensetzung auf die Gesichtshaut aufgetragen wird, so kann die Haut das Fett und/oder Öl nicht oder nicht vollständig aufnehmen, so dass ein feiner Fettfilm auf der Oberfläche bleibt. Die in der Zusammensetzung enthaltenen Pigmente schwimmen in diesem Fettanteil, der sich auf der Haut bewegen kann und dazu neigt, in Vertiefungen, wie Fältchen und Runzeln zu wandern. Dadurch kommt es zu einer Ansammlung der Pigmente in Fältchen, Poren, Vertiefungen und ähnlichen Unregelmäßigkeiten, was diese betont. Dies ist unerwünscht, da gerade Poren, Falten und Ungleichmäßigkeiten verdeckt werden sollen

Ein weiteres Problem besteht darin, dass die auf einen Bereich aufgetragene Zubereitung entweder von dem aufgetragenen Ort wegwandert, was zu einem unästhetischen Aussehen führt, oder aber an damit in Berührung kommenden Gegenständen oder Haut, z.B. der Hand, haftet und diese verfärbt. Beides ist unerwünscht.

Weiterhin ist aus EP-A 0 793 957 eine kosmetische Zusammensetzung bekannt, die ein filmbildendes Polymer in wässriger Dispersion enthält. Pigmente können dieser Zusammensetzung zugemischt werden. Mit Hilfe einer derartigen Dispersion, die insbesondere zur Verwendung als Lippenstift vorgesehen ist, kann eine gefärbte Schicht auf die Haut aufgebracht werden. Allerdings dauert es ziemlich lange, bis der Film getrocknet ist und sich damit nicht mehr klebrig oder ölig anfühlt, sondern leicht und kaum spürbar ist.

Aufgabe der Erfindung war es nun, eine kosmetische Zusammensetzung bereitzustellen, die auf die Haut oder Schleimhaut aufgetragen werden kann, durch ihre Zusammensetzung Falten verdeckt oder kaschiert, am aufgetragenen Ort haften bleibt und nicht wegwandert und sich auch nicht auf Gegenstände, mit denen die Zusammensetzung in Berührung kommt, überträgt. Darüber hinaus soll ein Produkt bereitgestellt werden, das sehr schnell nach dem Auftragen die gewünschte Wirkung erzielt und sich nicht klebrig anfühlt.

Diese Aufgabe wird gelöst mit einer Zubereitung in Form einer Emulsion zum Auftragen auf Haut und Schleimhaut, die eine wässrige Phase mit einer filmbildenden Polymer-Kombination aus mindestens zwei Polymeren und eine emulgierte Phase mit mindestens einem flüchtigen Silikon, mindestens einem Pigment und ggf. Füllstoffen aufweist, wobei die filmbildende Polymerkombination in Form einer wässrigen Lösung oder Dispersion eingesetzt wird, wobei die filmbildende Polymerkombination ein elastisches Polymer und ein Festigkeit oder Zähigkeit vermittelndes Polymer aufweist und sie ferner in einem Anteil von 5 bis 50 Gew.-% bezogen auf die gesamte Zubereitung enthalten ist, und wobei als Pigment mindestens ein kugelförmiges feines Pigment in Form von LDP enthalten ist.

Überraschenderweise wurde festgestellt, dass es mit der erfindungsgemäßen Zubereitung möglich ist, einen lang haltbaren Film auf der Haut oder der Schleimhaut zu erzeugen, der der Trägerin ein sehr angenehmes Gefühl vermittelt, der am aufgetragenen Ort haftet, ohne von dort wegzuwandern, andererseits aber so elastisch ist, dass er alle Bewegungen der Haut oder Schleimhaut mitmacht, ohne sich zu lösen. Darüber hinaus bilden die Polymere der Zusammensetzung, einerseits einen Film, und kaschieren andererseits in Kooperation mit den Pigmenten und ggf. den Füllstoffen, Unebenheiten, Fältchen und Poren in der Haut. All diese Eigenschaften können erzielt werden, ohne dass natürliche Fette und Öle oder Wachse in der Zusammensetzung enthalten sind.

Die erfindungsgemäße Zubereitung hat die Form einer Zusammensetzung mit einer Konsistenz, die von fließfähig bis pastenartig reichen kann. Sie ist daher sowohl geeignet für fluide Make-ups, als auch cremeartige Produkte, wie Concealer, Camouflagecremes oder Theaterschminken. Die Zubereitung kann auf Haut, Schleimhaut oder auch Semi-Schleimhaut aufgetragen werden und ist vor allen Dingen vorgesehen zum Auftragen auf Gesicht und Hals, beispielsweise die Gesichtshaut, den Bereich um die Augen, die Lippen und die Umgebung der Lippen usw. Ein Auftragen auf Dekolleté, Oberarme und sonstige Körperbereiche, wo eine kaschierende Wirkung erzielt werden soll, ist ebenfalls möglich.

Die erfindungsgemäße Zubereitung basiert im Wesentlichen auf filmbildenden Substanzen, Färbemitteln und flüchtigen Substanzen, nämlich Wasser und mindestens einem flüchtigen Silikon.

Wenn im folgenden Prozentangaben für Inhaltsstoffe gemacht werden, so sind diese immer als Gewichtsprozent bezogen auf das Gesamtgewicht der Zubereitung zu verstehen, wenn nichts anderes angegeben ist.

Ein wichtiger Bestandteil der erfindungsgemäßen Zubereitung ist eine filmbildende Polymer-Kombination aus mindestens zwei Polymeren. Unter einer "filmbildenden Polymer-Kombination aus mindestens zwei Polymeren", wird im Rahmen der vorliegenden Erfindung eine Kombination aus zwei Polymeren verstanden, einem Polymerblend, das aus mindestens zwei verschiedenen Polymeren gebildet wird. Wesentlich ist, dass die Kombination einen elastischen und trotzdem festen oder zähen Film auf der Haut bilden kann.

Es hat sich herausgestellt, dass es zur Erzielung eines Films mit den gewünschten Eigenschaften notwendig ist, zwei verschiedene Polymerarten einzusetzen. Ein Polymer trägt zur Elastizität bei und das andere bewirkt die Festigkeit des Films. Dabei ist es erfindungsgemäß möglich, ein Polymerblend aus mindestens zwei verschiedenen Polymeren einzusetzen. Wichtig ist, dass der erhaltene Film flexibel aber fest und im Wesentlichen nicht ortsbeweglich ist.

Für die erfindungsgemäße Zubereitung wird eine filmbildende Polymerkombination aus einem elastischen Polymer und einem Festigkeit oder Zähigkeit vermittelnden Polymer verwendet. Als elastisches Polymer werden bevorzugt Polymere oder Copolymere von Acrylat oder Derivaten davon, Methacrylat oder Derivaten davon, Polyvinylalkohol und/oder Polyvinylacetat bzw. Copolymere aus zwei oder mehr der genannten Monomere oder Mischungen davon eingesetzt. Als das Festigkeit oder Zähigkeit vermittelnde Polymer werden besonders bevorzugt Polyurethane, Polyetherurethane, Polyesterurethane, Polyvinylpyrrolidon oder Mischungen davon verwendet.

Es wurde gefunden, dass diese Kombination von Eigenschaften dann erzielt wird, wenn entweder eine Mischung aus Polyurethanpolymer und Acrylat- bzw. Methacrylatpolymer oder -copolymer verwendet wird. Wenn hier und in den Ansprüchen ein Polymer als "Acrylat" bezeichnet wird, so sollen hierunter Polymere verstanden werden, die aus Acrylsäure oder deren Derivaten polymerisiert oder copolymerisiert wurden. So umfasst der Ausdruck "Acrylat" auch Acrylamide, Acrylester wie Acrylacetate etc, aber auch Copolymere, in denen das Acrylat das überwiegend vorhandene Polymer ist. Der Ausdruck "Polyurethan" umfasst generell Polymere, die Umsetzungsprodukte von bi- und polyfunktionellen Isocyanaten mit Hydroxylgruppen-haltigen Monomeren, wie Polyalkoholen, oder Polyestern und Polyethern enthalten.

Für die erfindungsgemäße filmbildende Polymerkombination kommen insbesondere solche Polymere oder Copolymere inbetracht, die von Acrylsäure oder Methacrylsäure und deren Derivaten, wie Amiden und Estern, insbesondere Acrylsäure, Acrylamiden und Acrylestern abgeleitet sind. Sie können ggf. basische Comonomere, wie primäre, sekundäre, tertiäre oder quarternäre Aminosubstituenten, z.B. Quarternisierungsprodukte von Dimethylaminoethylmethacrylat- oder Diallyldimethylammoniumacrylsäure-Anteilen; hydrophobe Substituenten, wie langkettige Alkylgruppen mit 10 bis 30, bevorzugt 12 bis 24 C-Atomen, saure Substituenten, wie Sulfonat-, Phosphat- und Carboxylgruppen oder hydroxylgruppenhaltige Anteile, wie Vinylalkohol, aufweisen. Auch Copolymere aus Vinylpyrrolidon/Acrylat oder Dialkylaminoalkylmethacrylat sind geeignet. Zu den Polyurethanen werden auch Polyurethanpolyvinylpyrrolidone, Polyester-Polyurethane, Polyether-Polyurethane sowie Polyharnstoffe gezählt. Das Polyurethan kann beispielsweise ein aliphatisches, cycloaliphatisches oder aromatisches Polyurethan-, Polyharnstoff/urethan oder Polyharnstoffcopolymer sein. Polyurethane können weiterhin aus Polyestern, die gradkettig oder verzweigt sind, oder Alkydverbindungen, die mobilen Wasserstoff aufweisen, hergestellt. Sie können auch saure oder basische Gruppen aufweisen.

Es sind viele kosmetisch annehmbare Polyurethane und Polyurethancopolymere bekannt, die die gewünschten Filme bilden und in wässriger Dispersion oder Lösung vorliegen.

Acrylpolymere können durch Polymerisation oder Copolymerisation von Acrylsäure-, Methacrylsäure-, Acrylsäureester-, Methacrylsäureester-, Acrylamid- und/oder Methacrylamidmonomeren entstanden sein. Als Beispiele für geeignete Monomere sind Methylmethacrylat, Ethylmethacrylat, Butylmethacrylat, Isobutylmethacrylat, 2-Ethylhexylmethacrylat und Laurylmethacrylat, N-tert.Butylacrylamid, N-tert.-Oktylacrylamid und Hydroxyalkylacrylate zu nennen. Diese können ggf. in Kombination mit Vinylmonomeren, wie Vinylestern, Vinylacetat, Vinylalkohol, Styrol oder Butadien copolymerisiert werden. Polymere und Copolymere aus Vinylpyrrolidon, ggf. in Kombination mit Acrylat oder Methacrylat, Polyvinylalkohohle und. Polyvinylacetate sind ebenfalls für die Erfindung geeignete Polymere. Auf Vinylpyrrolidon basierende Polymere oder polyurethanartige Polymere tragen zur Festigkeit des gebildeten Films bei, während die Acrylate, Methacrylate, Polyvinylalkohole und Polyvinylacetate die elastischen Eigenschaften beisteuern.

Um weitere Eigenschaften des Film zu beeinflussen, können auch weitere Monomere mit copolymerisiert werden bzw. als Blöcke in die Copolymere eingefügt werden, oder weitere Polymere den Polymerblends zugefügt werden. So ist es vorteilhaft, ein Polymer, das hydrophobe Eigenschaften verleiht, mit einzuarbeiten. Hier kommen Fettsäureether und -ester in Betracht. Als besonders geeignet haben sich Alkylsebacate und Stearylether und -ester erwiesen.

Besonders beständige, elastische Filme können mit den im Folgenden aufgeführten Polymeren und Copolymeren und insbesondere einer Kombination von mindestens zwei der genannten Polymere bzw. Copolymere erhalten werden (jeweils mit INCI-Namen angegeben): Octadecene/MA Copolymer/Diethylhexyl-Sebacate, Acrylates Copolymer, Polyurethane-Acrylates Copolymer, Lauryl Dimethicone, PVP/Dimethyl Aminoethylmethacrylate/Polycarbamyl/Polyglycolester, PVP/Dimethiconylacrylate/Polycarbamyl/Polyglycolester, PPG/IPDI/DPMA Crosspolymer, Alkylacrylate copolymer, Acrylates/Stearyl Acrylate/Dimethicone Methacrylate Copolymer, Polyvinyl Stearyl Ether, Acrylates/Lauryl Acrylate/Stearyl Acrylate/Ethylamine Oxide Methacrylate Copolymer.

Der Anteil der jeweils eingesetzten Polymere wird so ausgewählt, dass die gewünschten Eigenschaften erzielt werden. Dies kann durch wenige Routineversuche festgestellt werden. Geeignet sind z.B. solche Polymerblends, die Filme bilden, die bei Körpertemperatur eine Reißdehnung von 150% oder mehr, bevorzugt von 200 % oder mehr aufweisen. Die Reißdehnung kann gemäß der Norm ASTM-D2370-92 bestimmt werden.

Die Polymere bzw. Polymerblends werden in Form einer Lösung und/oder Dispersion auf Wasserbasis eingesetzt. Derartige Lösungen und Dispersionen sind im Handel erhältlich. Der Feststoffgehalt dieser Lösungen bzw. Dispersionen liegt üblicherweise in einem Bereich von 20 bis 60 %, bevorzugt 30 bis 50%.

Die filmbildende Polymerkombination ist in der erfindungsgemäßen Zubereitung in einem solchen Anteil enthalten, dass sich nach dem Auftragen auf die Haut oder Schleimhaut ein ausreichend stabiler Film bilden kann. Dazu wird die Polymerlösung oder -dispersion (d.h. die die filmbildende Polymerkombination enthaltende wässrige Lösung oder Dispersion) in einem Anteil von 5 bis 50 Gew-% bezogen auf Gewicht der Zubereitung zugegeben. Bevorzugt werden 8 bis 35 Gew.-%, bevorzugter 5 bis 25 Gew-% Polymerlösung bzw. -dispersion verwendet und besonders bevorzugt 8 bis 18 Gew.-%.

Der aus der erfindungsgemäßen filmbildenden Polymerkombination gebildete Film fixiert die weiteren Bestandteile und hält sie auf der Haut fest, bis er wieder gelöst wird.

Die wässrige Phase der erfindungsgemäßen Zubereitung wird aus der filmbildenden Polymerkombination, wie oben definiert und ggf. einem weiteren Anteil eines wässrigen Mediums, bevorzugt Wasser, gebildet. Wie oben ausgeführt, sind Polymerlösungen und -dispersionen im Handel erhältlich, die dann bei Verwendung in der erfindungsgemäßen Zubereitung noch mit Wasser "verdünnt" werden können, falls dies notwendig erscheint. Der erfindungsgemäßen Zubereitung kann daher abhängig von der gewünschten Viskosität und dem gewünschten Wassergehalt sowie abhängig von dem Wassergehalt der eingesetzten Polymerlösung oder Polymerdispersion noch wässriges Medium, insbesondere Wasser zugefügt werden, wobei der Anteil an Wasser nicht kritisch ist und nur durch die gewünschten Eigenschaften bestimmt wird. In der Regel liegt der zusätzliche Anteil an Wasser, d.h. der Anteil an Wasser, der zusätzlich zu dem in der Polymerdispersion oder Polymerlösung enthaltenen Wasseranteil zugefügt wird, in einem Bereich von 0 bis 60%, bevorzugt 20 bis 50%.

Ein weiterer wesentlicher Bestandteil der erfindungsgemäßen Zubereitung ist der Anteil an färbenden und/oder farblosen Pigmenten, der für die ästhetische Wirkung sorgt_Unter "Pigment" sollen im Rahmen der vorliegenden Erfindung nicht nur die klassischen anorganischen Pigmente verstanden werden, sondern generell farblose, weiße oder farbige anorganische und organische Pigmente, aber auch Farblacke, Perglanzmittel und sog. "Light diffusing pigments" oder "LDP" und Verlackungen organischer Farbstoffe verstanden werden. Weiterhin sind unter diesen Bestandteil auch Metallpulver und weiße oder farblose Pulver, wie Mica oder Kaolin zu subsumieren.

Mindestens ein Pigment muss in der erfindungsgemäßen Zubereitung enthalten sein, wobei dieses ein LDP oder lichtreflektierendes Pigment ist. Weitere Pigmente für besondere Effekte oder zur Farbgebung können hinzukommen. Beispielhaft genannt werden können als anorganische Pigmente z.B. gelbe, schwarze oder rote Eisenoxide, Ultramarine, Chromoxidgrün, Chromoxydhydratgrün, Ruß (Carbon Black); organische Pigmente, Verlackungen organischer Farbstoffe, Karmin, plättchenförmige Metallpulver, wie passiviertes Aluminium, Messing, Bronze, Kupfer, Silber oder Gold; Glimmer, mit Metalloxiden, z.B. mit Titandioxid, Eisenoxiden, Chromoxid, Chromoxidhydrat beschichtete Glimmer; plättchenförmige Zubereitungen auf Basis von Siliciumdioxid, Aluminuimoxid oder Glas, die ggf. auch mit Metalloxiden, Titandioxid, Eisenoxid, Chromoxid, Chromoxidhydrat beschichtet sein können, und deren Mischungen.

Die Pigmente werden üblicherweise in sehr feinteiliger Form verwendet, damit sie sich gut verteilen und eine gleichmäßige Wirkung erzielen. Ggf. können die Pigmente so beschichtet oder derivatisiert sein, dass sie mit den anderen Bestandteilen besonders gut kompatibel sind. Verfahren zur Bearbeitung von Pigmenten und zu ihrer Derivatisierung sind dem Fachmann bekannt.

Die Pigmente werden je nach der gewünschten Wirkung und je nach dem gewünschten Färbungsgrad in einem Anteil von 0,1 bis 25 Gew.-%, bevorzugt 0,1 bis 15 Gew-%, bezogen auf die Gesamtzusammensetzung, eingesetzt. Falls eine stärkere Färbung erwünscht ist, kann auch ein höherer Anteil an Pigment verwendet werden. In diesem Fall ist darauf zu achten, dass eine ausreichende Stabilisierung gewährleistet ist.

Erfindungsgemäß werden sowohl Zubereitungen in Betracht gezogen, die nur zum Kaschieren der Falten vorgesehen sind und daher keine farbigen Pigmente, sondern nur LDP enthalten, als auch Make-up-Produkte, die einen eigenen Farbton haben und auf der Haut einen farbigen Film bilden. Die erfindungsgemäße Zubereitung kann auch in Form von Camouflageprodukten oder auch Theaterschminken eingesetzt werden, wobei in diesen Fällen der Anteil an Pigmenten eher im oberen Bereich liegt. Wenn die erfindungsgemäße Zubereitung nur dazu verwendet werden soll, Fältchen rund um die Augen oder um den Mund "unsichtbar" zu machen, wird der Pigmentanteil im unteren Bereich ausgewählt.

Um die Pigmente stabil in der erfindungsgemäßen Zubereitung zu verteilen und dispergiert zu halten, ist ein Träger notwendig. Dieser Träger ist ein weiterer wichtiger Bestandteil der erfindungsgemäßen Zubereitung und ist ein flüchtiges Silikon, das als "Träger" für das Pigment und ggf. weitere Bestandteile dient. Als "flüchtiges Silikon" werden Silikone mit eher niederem Molekulargewicht bezeichnet, die bei der Temperatur der Haut relativ schnell verdampfen.. Insbesondere werden solche Silikone in Betracht gezogen, deren Flammpunkt unter 50° C, bevorzugt unter 40° C liegt, mit entsprechender Abdampfkinetik. Flüchtige Silikone werden für kosmetische Produkte häufig eingesetzt und die kosmetisch annehmbaren Produkte sind für die vorliegende Erfindung geeignet.

Derartige Silikone sind dem Fachmann wohlbekannt. Als Beispiele können cyclische Dimethicone bzw. Cyclomethicone mit 3 bis 7 Si-Einheiten, lineare Dimethlylsiloxane, Dimethicone oder Methicone oder deren Derivate, z.B. solche mit bis zu 100, bevorzugt 2 bis 20 Siloxaneinheiten, die an den Enden und/oder in der Kette Alkylreste mit 1 bis 6 C-Atomen und/oder Phenylreste aufweisen können, und insbesondere Dimethicone, Hexamethyldisiloxan, Cyclomethicone, Dodecamethylcyclohexasiloxan, Decamethylacyclopentasiloxan, Octamethylcyclotetrasiloxan, und Derivate davon sowie Mischungen der genannten Bestandteile genannt werden. Ggf. können weitere flüchtige Verbindungen zugefügt werden, die mit den Silikonen kompatibel sind, z.B. Ethyl Perfluoroisobutylether oder Ethyl Perfluorobutyl Ether.

Die erfindungsgemäße Zubereitung kann weiterhin Füllstoffe enthalten, die dazu dienen, in die Hautvertiefungen, Fältchen, Poren etc. zu wandern, diese "aufzufüllen" und dadurch ein glatteres Hautbild zu schaffen. Diese Füllstoffe können außerdem beim Auftragen und nach dem Auftragen auf der Haut am aufgetragenen Ort ein angenehmes Gefühl vermitteln. Als Füllstoffe werden bevorzugt anorganische oder organische feinteilige Pulver verwendet. Beispiele sind Dimethicone/Vinyldimethicone-Crosspolymer, Polyamide, Polyethylene, Kaolin, Talkum, Boron Nitride, PMMA, Methylmethacrylate-Crosspolymer, Silica, Alumina, Mica, PTFE, Lauroyllysin und andere. Auch eine Mischung dieser Stoffe kann nach Bedarf eingesetzt werden.

Der Anteil der Füllstoffe wird so eingestellt, dass die dynamische Viskosität der Zubereitung in einem geeigneten Bereich liegt und die gewünschte Wirkung erzielt wird. Als geeignet haben sich Anteile im Bereich von 0,5 bis 10 Gew-%, bevorzugt 1,5 bis 8 Gew-% erwiesen.

Die erfindungsgemäße Zubereitung liegt in Form einer Emulsion vor, wobei in Silikon dispergierte Pigmente und Füllstoffe die emulgierte Phase und eine wässrige Lösung oder Dispersion von filmbildenden Polymeren ggf. mit zugefügtem wässrigem Medium die kontinuierliche Phase bildet. Um die Emulsion zu erzeugen und stabil zu halten, können ein oder mehrere Emulgatoren verwendet werden. Dabei ist es bevorzugt, nicht-ionogene Emulgatoren einzusetzen, um die Eigenschaften verändernde Reaktionen zwischen Emulgator und kationischen oder anionischen Bestandteilen zu vermeiden. Ein weiterer Vorteil der vorliegenden Erfindung besteht darin, dass es möglich ist, auf die Verwendung von polyethylenglycolhaltigen oder polypropylenglycolhaltigen Emulgatoren zu verzichten, die unangenehme Nebenwirkungen haben können. Für die erfindungsgemäßen Zubereitungen haben sich als geeignet auf Potyhydroxyverbinduhgen, Zucker- und Zuckerderivaten oder Fettsäuren basierende Emulgatoren erwiesen. Besonders geeignet sind Polyglyceryl-3 Methylglucose-Distearate, Perfluorononyl Dimethicone und Derivate, Alkyl Dimethicone, Acrylates/Stearyl Acrylate/Dimethicone Methacrylat Copolymer, Hydroxypropyl Dimethicone Behenat, Dimethicone Propyl PG-Betaine, Polyglyceryl-4-Isostearate, Glyceryl Stearate, Glyceryl Stearate Citrate, Caprylyl Dimethicone Ethoxyglucoside.

Die Emulgatoren werden, falls verwendet, bevorzugt in einem Anteil von 3 bis 10 %, bezogen auf die Gesamtzusammensetzung, zugegeben.

Wie oben ausgeführt, kann die erfindungsgemäße Emulsion eine Konsistenz von fließfähig bis pastenartig haben. Um die für die jeweilige Form geeignete Viskosität einzustellen, kann der Zusammensetzung ein Verdicker zugesetzt werden. Als Verdicker kommen alle kosmetisch annehmbaren Substanzen in Betracht, die eine verdickende Wirkung in Gegenwart von Wasser aufweisen, mit den filmbildenden Polymeren und anderen Inhaltsstoffen kompatibel sind und stabile Produkte liefern. In der Regel werden natürliche oder synthetische Polymere für diesen Zweck verwendet. Beispiele sind Hydrokolloide, wie Xanthangummi, Gummi arabicum, Johannisbrotkernmehl; Gelatine; polymere Verdicker, wie z.B. Polyacrylate und die unter der Bezeichnung "Carbopol^{®}" vertriebenen Produkte usw. Der Anteil des Verdickers ist abhängig von der gewünschten Viskosität und liegt üblicherweise in einem Bereich von 0 bis 5 Gew-%.

Die dynamische Viskosität der erfindungsgemäßen Emulsion ist abhängig von der gewünschten Konsistenz, liegt aber allgemein in einem Bereich von 3 bis 50 Pa•s, vorzugsweise 10 bis 25 Pa•s, gemessen mit einem Brookfield-Viskosimeter mit Spindel 4 bei 60 Umin⁻¹.

Ein weiterer, fakultativer Bestandteil, der für einen auf der Haut angenehmen Film sorgen soll, ist ein Feuchthaltemittel, das nach Bedarf eingesetzt werden kann. Feuchthaltemittel sind hygroskopische Verbindungen, in der Regel hydroxylgruppenhaltige Verbindungen. Als Beispiele können Glycole, wie 1,2-Propylenglycol, Dipropylenglycol, Tripropylenglycol, Glycerin, Diglycerin, Triglycerin, Butylenglycol, Hexylenglycol; Zucker und Zuckeralkohole, wie Mannitol, Sorbitol, Xylitol, Glucose, Fructose, Saccharose, Maltose; Harnstoff, Milchsäure und ihre Salze, z.B. Natriumlactat, Pyrrolidoncarbonsäure, deren Natriumsalz und Mischungen davon genannt werden. Falls verwendet, wird das Feuchthaltemittel in der Regel in einem Anteil von 0,1 bis 5 Gew-% bezogen auf die Gesamtzusammensetzung eingesetzt.

Der pH-Wert der erfindungsgemäßen Emulsion sollte im Wesentlichen im neutralen Bereich liegen, um die Haut nicht zu reizen. Üblicherweise haben kosmetische Produkte einen pH-Wert im Bereich zwischen 5 und 8,5, bevorzugt 5,5 und 8,0 besonders bevorzugt 7,2 bis 8,0, was dem Bereich der Tränenflüssigkeit entspricht. Ggf. kann der geeignete pH-Wert in üblicher Weise eingestellt werden durch Zugabe von sauren oder basischen Substanzen, wie sie allgemein auf dem Gebiet der Kosmetik üblich sind.

Da die erfindungsgemäße Zubereitung einen hohen Anteil an Wasser enthält, kann es vorteilhaft sein, ein Konservierungsmittel zu verwenden, um einen mikrobiellen Befall zu verhindern. Für Kosmetika geeignete Konservierungsmittel sind dem Fachmann wohlbekannt und jedes geeignete kann eingesetzt werden. Der Anteil der Konservierungsmittel liegt im üblichen Bereich und übersteigt in der Regel 1 Gew-% bezogen auf die Zusammensetzung nicht.

Weitere Bestandteile, die der erfindungsgemäßen Zusammensetzung zugesetzt werden können, um bestimmte Effekte zu erzielen, sind Antioxidantien, pflanzliche oder tierische Extrakte, Riechstoffgemische, Lichtschutzfiiter und dergleichen. All diese Mittel sind dem Fachmann wohlbekannt und die in der Kosmetik allgemein verwendeten können hier in den üblicherweise verwendeten Mengen eingesetzt werden. Ihr Anteil liegt im üblichen Bereich und übersteigt in der Regel 5 Gew-%, bevorzugt 1 Gew-%, bezogen auf die Zusammensetzung nicht.

Die erfindungsgemäße Zubereitung hat eine sehr angenehme Konsistenz und lässt sich leicht auf die Haut auftragen. Nach dem Auftragen verdampfen die flüchtigen Anteile und lassen einen elastischen angenehmen Film auf der Haut zurück. Die enthaltenen Füllstoffe und Pigmente und teilweise auch die eingesetzten Polymere setzen sich in Vertiefungen, wie Poren und Falten, ab und füllen diese dadurch auf. Der elastische Film sorgt dafür, dass die Hautoberfläche gestrafft wird. Auf diese Weise erhält die Haut ein optisch verbessertes, glattes Aussehen. Der gebildete Film weist eine reduzierte Migration und Nontransfer-Eigenschaften auf. Er ist beständig gegen Sebum und bedingt beständig gegen Wasser. Mit warmem Wasser und ggf. etwas Reiben kann der auf die Haut aufgetragene Film jedoch leicht wieder entfernt werden.

In die erfindungsgemäße Zubereitung können darüber hinaus, falls dies erwüscht ist, auch noch Wirkstoffe eingearbeitet werden, die der Haut beruhigende, entzündungshemmende und ähnliche Eigenschaften vermitteln sollen. Hierzu sind wasserlösliche pflanzliche und tierische Wirkstoffe besonders gut geeignet. Zu nennen sind beispielsweise Bisabolol, Pantothenol, wässrige Extrakte von Calendula, Kamille, Schafgarbe, Fenchel, Johanniskraut und andere Extrakte, die auf der Haut angenehm sind.

Ein weiterer Gegenstand der vorliegenden Erfindung ist ein Verfahren zur Herstellung der oben beschriebenen Zubereitung. Dazu werden Pigmente und, falls verwendet, Füllstoffe in einem flüchtigen Silikon verteilt oder dispergiert, bevorzugt durch Homogenisieren. Es wird weiterhin eine wässrige Phase bereitgestellt, die ggf. Konservierungsmittel, Feuchthaltemittel und andere wasserlösliche Bestandteile, ggf. unter Ausnahme der filmbildenden Polymerkombination, enthält. Falls ein Emulgator verwendet wird, kann dieser ebenfalls einer der beiden Phasen vor der Emulgierung zugesetzt werden. Falls der Emulgator eine wachsartige Konsistenz hat und oberhalb Raumtemperatur schmilzt, werden die beiden Phasen auf die entsprechende Temperatur, bei der der Emulgator schmilzt, gebracht.

Wenn der Emulgator bei Raumtemperaturen aktiv ist, kann die Emulgierung bei Raumtemperatur erfolgen. In einer Ausführungsform des erfindungsgemäßen Verfahrens werden Silikonphase und wässrige Phase miteinander vermischt und homogenisiert, um die Emulsion zu bilden. Danach wird die Lösung oder Dispersion der filmbildenden Polymere zugesetzt und nach Vermischen kann die Emulsion abgefüllt werden. In einer anderen Ausführungsform, falls die Verarbeitung bei Raumtemperatur erfolgt und die Polymere durch die Homogenisierung nicht beeinträchtigt werden, können die filmbildenden Polymere bereits vor der Emulgierung der wässrigen Phase zugesetzt werden und die Emulgierung danach erfolgen.

Die entstehende Emulsion ist stabil und kann, abgefüllt in Behältnissen, über längere Zeit aufbewahrt werden. Die erfindungsgemäß in dem flüchtigen Silikon dispergierten Pigmente und Füllstoffe werden in den Silikontröpfchen, die die emulgierte Phase bilden, stabil in der wässrigen Emulsion gehalten und setzen sich nicht ab.

Die erfindungsgemäße Zubereitung kann in Form einer Si/W-Emulsion oder W/Si/W-Emulsion vorliegen. Wesentlich ist, dass die kontinuierliche Phase eine wässrige Phase ist, in der die Silikonphase emulgiert ist. Durch diesen Aufbau und den Anteil an filmbildender Polymerkombination kann auf üblicherweise verwendete Fette, Wachse und Öle (in Form von Triglyceriden, flüssigen Fettsäureestern oder mineralischen Ölen), wie sie sonst notwendig sind, verzichtet werden. -

Da die erfindungsgemäße Kombination aus Filmbildnern einen weichen, angenehmen aber doch stabilen Film bildet, ist eine Verwendung von Wachsen nicht notwendig und die damit verbundenen Probleme werden dadurch vermieden. Auch Fette und Öle sind in den erfindungsgemäßen Emulsionen nicht vorhanden.

Da das Silikon als Träger für Pigmente und Farbstoffe sowie Füllstoffe dient, die äußere Phase aber von Wasser und darin gelösten oder dispergierten filmbildenden Polymeren gebildet wird, kann sich beim Auftragen ein Film bilden, der die Pigmente und Füllstoffe einschließt, so dass sie nach Verdampfung von Silikon und Wasser in dem Film fest verankert sind und nicht aus dem Film auswandern und in Fältchen einwandern können.

Der Verdicker kann entweder der Wasserphase vor der Emulgierung zugegeben werden, insbesondere wenn er vor der Emulgierung quellen muss. Er kann aber auch zusammen mit dem filmbildenden Polymer nach Fertigstellung der Emulsion zugesetzt werden.

Wie oben ausgeführt, bewirken einerseits in der Emulsion enthaltene Pigmente und gegebenenfalls Füllstoffe, dass Fältchen und Vertiefungen in der Haut aufgefüllt und optisch kaschiert werden, LDPs bewirken, dass Vertiefungen optisch aufgehellt werden und der aus den Filmbildnern gebildete Film bewirkt eine Straffung der Haut, so dass insgesamt ein sehr ebenes ästhetisches Hautbild erzeugt wird, ohne das Mikrorelief der Haut zu zerstören. Gleichzeitig ist der entstehende Film beständig gegen Schweiß und Tränen und überträgt sich nicht auf damit in Berührung kommende Gegenstände. Da die Zubereitung auch beliebig eingefärbt werden kann, eignet sie sich ausgezeichnet als unpigmentierter Linefiller ebenso wie als pigmentiertes Make-up, als Camouflage oder Theaterschminke, aber auch als fältchenkaschierende straffende Foundation.

Ein weiterer Gegenstand der Erfindung ist daher auch die Verwendung einer Emulsion mit einer wässrigen Phase, die eine filmbildende Polymerkombination enthält und einer emulgierten Phase, die ein oder mehrere flüchtige Silikone, mindestens ein Pigment und ggf. Füllstoffe enthält, zur Bildung einer faltenkaschierenden Schicht auf Haut und/oder Schleimhaut. Insbesondere ist Gegenstand der Erfindung die Verwendung einer Zubereitung, wie sie oben beschrieben wurde, zur Herstellung einer kosmetischen Zusammensetzung mit faltenkaschierenden Eigenschaften.

Besonders geeignet ist die erfindungsgemäße Zubereitung als Make up, Foundation, Concealer, Augencreme oder Lippencreme mit faltenkaschierender Wirkung. Bevorzugte Produkte werden verwendet, um Falten im Gesicht, um die Augen, um die Lippen und/oder am Hals zu kaschieren.

Die Erfindung wird anhand der nachfolgenden Beispiele noch näher erläutert.

Die Mengenangaben sind in Gew.-%, bezogen auf das Gesamtgewicht der Zubereitung. Die Rohstoffnamen entsprechen den dem einschlägig befassten Fachmann bekannten "INCI-Namen" (international Nomenclature of Cosmetic Ingredients).

### Beispiel 1 (Foundation für Gesicht und Hals)

| | | |
|---|---|---|
| A | Dimethicone/Vinyl Dimethicone Crosspolymer | 3,300 |
| | Dimethicone | 9,300 |
| | Decamethylcyclopentasiloxane | 12,300 |
| | Stearyl Dimethicone | 3,800 |
| | Polyglyceryl-3 Methylglucose Distearate | 2,500 |
| | | |
| B | Titanium Dioxide (C.I.-No. 77.891) | 4,800 |
| | Yellow Iron Oxide (C.I.-No. 77.492) | 1,200 |
| | Red Iron Oxide (C.I.-No. 77.491) | 1,200 |
| | Black Iron Oxide (C.I.-No. 77.499) | 0,800 |
| | Silica and Titanium Dioxide and Iron Oxides | 1,700 |
| | | |
| C | Aqua (destilliert) | 40,300 |
| | Sorbitol | 4,500 |
| | Diazolidinyl Urea | 0,200 |
| | Triethanolamine | 0,100 |
| | | |
| D | Octadecene/MA Copolymer and Diethylhexyl Sebacate* | 3,000 |
| | Acrylates Copolymer* | 2,500 |
| | Polyurethane-4* | 5,000 |
| | (*die Mengenangaben beziehen sich jeweils auf die Polymerdispersion) | |
| | | |
| E | Bentonite | 3,500 |

Die Bestandteile der Phase A werden in ein Gefäß eingebracht und auf 65°C erwärmt, bis eine klare Lösung entstanden ist, dann in eine Homogenisiermaschine eingeführt. Die Phase B wird zugegeben und die Mischung mittels eines Schnellrührers so lange gemischt, bis sie homogen ist. In einem separaten Gefäß wird die Phase C auf 65°C gebracht und anschließend unter leichtem Vakuum in die Mischung aus den Phasen A und B eingesaugt.

Danach wir die Mischung unter leichtem Vakuum 3-4 Minuten homogenisiert und dann auf etwa 40°C abgekühlt, dann wird nochmals 2 Minuten homogenisiert. Der Mischung wird jetzt die Phase D zugesetzt, etwa 2 Minuten intensiv gerührt, jedoch nicht mehr mittels Schnellrührers homogenisiert und jetzt das Bentonite in die Mischung eingestreut. Man rührt kurz mit dem Rührwerk um, dann wird etwa 10 Minuten mit dem Rührwerk nachgerührt und in dieser Phase maximales Vakuum angelegt, um den Ansatz zu entlüften. Es ist darauf zu achten, dass der pH-Wert nicht unter 7,1 absinkt, ggf. ist Triethanolamin zuzugeben. Der pH-Wert der fertigen Zubereitung soll bei 7,2 bis 7,7 und bevorzugt bei 7,5 liegen. Man erhält eine leicht hellbraune Zubereitung mit einer Viskosität von 5,5 bis 7,5 Pas (Nullviskosität), welche sich insbesondere zur Abfüllung in Auftraggeräte eignet, wie sie beispielsweise in US 6,309,128 oder US 6,238,117 beschrieben sind. Selbstverständlich kann diese Zubereitung-auch in üblicher Weise in geeignete Behältnisse abgefüllt werden.

### Beispiel 2 (pastöser Concealer)

| | | |
|---|---|---|
| A | Dimethicone/Vinyl Dimethicone Crosspolymer | 3,300 |
| | Dimethicone | 10,300 |
| | Decamethylcyclopentasiloxane | 12,300 |
| | Stearyl Dimethicone | 3,800 |
| | Polyglyceryl-3 Methylglucose Distearate | 2,500 |
| | | |
| B | Titanium Dioxide (C.I.-No. 77.891) | 1,300 |
| | Yellow Iron Oxide (C.I.-No. 77.492) | 0,600 |
| | Red Iron Oxide (C.I-No. 77.491) | 0,800 |
| | Black Iron Oxide (C.I.-No. 77.499) | 0,250 |
| | Mica (C.I. -No. 77.891) | 3,500 |
| | Silica and Titanium Dioxide and Iron Oxides | 2,300 |
| | | |
| C | Aqua (destilliert) | 39,250 |
| | Sorbitol | 3,500 |
| | Diazolidinyl Urea | 0,200 |
| | Triethanolamine | 0,100 |
| | | |
| D | Octadecene/MA Copolymer and Diethylhexyl Sebacate* | 3,000 |
| | Acrylates Copolymer* | 3,500 |
| | Polyurethane-4* | 5,000 |
| | (*die Mengenangaben beziehen sich jeweils auf die Polymerdispersion) | |
| | | |
| E | Bentonite | 4,500 |

Die Herstellung erfolgt analog zu Beispiel 1. Man erhält eine relativ transparente, fast hautfarbene Zubereitung mit leichtem Glanz, welche die Feinfältelung der Haut, insbesondere im Bereich um die und unter den Augen, sehr gut überdeckt. Diese Zubereitung kann tel quel auf der Haut verbleiben - sie kann aber auch nach dem Trocknen als Basis eines Make-up dienen. Sie weist eine Viskosität im Bereich 7 bis 12 Pas auf.

## Patentansprüche

1. Zubereitung in Form einer Emulsion zum Auftragen auf Haut und/oder Schleimhaut, enthaltend eine wässrige Phase mit einer filmbildenden Polymerkombination aus mindestens zwei Polymeren und eine emulgierte Phase mit mindestens einem flüchtigen Silikon, mindestens einem Pigment sowie ggf. Füllstoffen, wobei die filmbildende Polymerkombination in Form einer wässrigen Lösung oder Dispersion eingesetzt wird, wobei die filmbildende Polymerkombination ein elastisches Polymer und ein Festigkeit oder Zähigkeit vermittelndes Polymer aufweist und sie ferner in einem Anteil von 5 bis 50 Gew.-% bezogen auf die gesamte Zubereitung enthalten ist, und wobei als Pigment mindestens ein kugelförmiges feines Pigment in Form von LDP enthalten ist.

2. Zubereitung nach Anspruch 1, **dadurch gekennzeichnet, dass** das elastische Polymer ein Acrylat oder ein Derivat davon, ein Methacrylat oder ein Derivat davon, ein Polyvinylalkohol und/oder ein Polyvinylacetat oder ein Copolymer aus zwei oder mehreren der genannten Monomere oder eine Mischung davon ist.

3. Zubereitung nach Anspruch 1 oder 2, **dadurch gekennzeichnet, dass** das Festigkeit oder Zähigkeit vermittelnde Polymer ein Polyurethan, Polyetherurethan, Polyesterurethan, Polyvinylpyrrolidon oder eine Mischung davon ist.

4. Zubereitung nach einem der vorhergehenden Ansprüche, **dadurch gekennzeichnet, dass** die filmbildende Polymerkombination ein Polyurethan-Acrylat-Copolymer enthält.

5. Zubereitung nach einem der vorhergehenden Ansprüche, **dadurch gekennzeichnet, dass** die filmbildende Polymerkombination ein Polymerblend aus einem Polyurethanpolymer und/oder Polyvinylpyrrolidonpolymer in Kombination mit einem Acrylat-, Acrylamid-, Acrylesterpolymer, einem Polyvinylalkohol und/oder Polyvinylacetat ist.

6. Zubereitung nach Anspruch 1, **dadurch gekennzeichnet, dass** die Polymerkombination ausgewählt wird aus Octadecene/MA Copolymer/Diethylhexyl-Sebacate, Acrylates Copolymer, Polyurethane-Acrylates Copolymer, Lauryl Dimethicone, PVP/Dimethyl Aminoethylmethacrylate/Polycarbamyl/Polyglycolester, PVP/Dimethiconylacrylate/Polycarbamyl/Polyglycolester, PPG/IPDI/DPMA Crosspolymer, Alkylacrylate Copolymer, Acrylates/Stearyl Acrylate/Dimethicone Methacrylate Copolymer, Polyvinyl Stearyl Ether, Acrylates/Lauryl Acrylate/Stearyl Acrylate/Ethylamine Oxide Methacrylate Copolymer, Polyurethane und deren Mischungen.

7. Zubereitung nach einem der vorhergehenden Ansprüche, **dadurch gekennzeichnet, dass** als filmbildende Polymerkombination eine Kombination aus einem oder mehreren Polyurethanen und einem oder mehreren Acrylaten und/oder Methacrylaten in Form eines Polymerblends vorhanden ist.

8. Zubereitung nach einem der vorhergehenden Ansprüche, **dadurch gekennzeichnet, dass** eine Kombination von filmbildenden Polymeren ausgewählt aus Polymeren, Copolymeren und/oder Crosspolymeren von Polyvinylpyrrolidon, Polyvinylalkohol, Polyvinylacetat, Polyurethan, Polyacrylat und Polymethacrylat enthalten ist.

9. Zubereitung nach einem der vorhergehenden Ansprüche, **dadurch gekennzeichnet, dass** die filmbildende Polymerkombination in einem Anteil von 5 bis 25 Gew.-% bezogen auf die gesamte Zubereitung enthalten ist.

10. Zubereitung nach einem der vorhergehenden Ansprüche, **dadurch gekennzeichnet, dass** in der wässrigen Phase zusätzlich mindestens ein Feuchthaltemittel enthalten ist.

11. Zubereitung nach Anspruch 5, **dadurch gekennzeichnet, dass** als Feuchthaltemittel 1,2-Propylenglycol, Dipropylenglycol, Tripropylenglycol, Glycerin, Diglycerin, Triglycerin, Butylenglycol, Hexylenglycol, Zucker, Zuckeralkohol, Mannitol, Sorbitol, Xylitol, Glucose, Fructose, Sucrose, Maltose, Harnstoff, Milchsäure in Na-Lactat, Sodium-PCA oder ein Gemisch daraus enthalten ist.

12. Zubereitung nach einem der vorhergehenden Ansprüche, **dadurch gekennzeichnet, dass** zusätzlich zu LDP ein oder mehrere Pigmente ausgewählt aus organischen Pigmenten, anorganischen Pigmenten, Verlackungen organischer Farbstoffe, Metallpulver, beschichtete Metallpulvern, plättchenförmigen Zubereitungen auf Basis von Siliciumdioxid, Aluminiumoxid oder Glas oder anderen Mischungen enthalten sind.

13. Kosmetische Zubereitung nach Anspruch 1, **dadurch gekennzeichnet, dass** das flüchtige Silikon Dimethicone, Hexamethyldisiloxan, Cyclomethicone, Dodecamethylcyclohexasiloxan, Decamethylcyclopentasiloxan, Octamethylcyclotetrasiloxan, oder ein Derivat oder eine Mischung daraus ist, ggf. in Kombination mit Ethyl Perfluoroisobutyl Ether oder Ethyl Perfluorobutyl Ether.

14. Zubereitung nach einem der vorhergehenden Ansprüche, **dadurch gekennzeichnet, dass** ein Füllstoff enthalten ist.

15. Zubereitung nach einem der vorhergehenden Ansprüche, **dadurch gekennzeichnet, dass** als Füllstoff DimethiconelVinyldimethicone Crosspolymer, Polyamid, Polyethylen, Kaolin, Talkum, Boronnitrid, PMMA, Methylmethacrylatcrosspolymer, Silica, Alumina, Mica, PTFE, Lauroyllysin enthalten ist.

16. Zubereitung nach einem der vorhergehenden Ansprüche, **dadurch gekennzeichnet, dass** zusätzlich ein Wirkstoff enthalten ist.

17. Zubereitung nach einem der vorhergehenden Ansprüche, **dadurch gekennzeichnet, dass** die Zubereitung wachs-, fett und ölfrei ist.

18. Zubereitung nach einem der vorhergehenden Ansprüche, **dadurch gekennzeichnet, dass** zusätzlich ein Emulgator enthalten ist.

19. Zubereitung nach einem der vorhergehenden Ansprüche, **dadurch gekennzeichnet, dass** ein nicht-ionogener Emulgator enthalten ist, der bevorzugt ausgewählt ist aus Polyglyceryl-3-Methylglucose-Distearat, Perfluorononyl-Dimethicone und Derivaten davon, Alkyldimethicone, Acrylate/Stearyl AcrylatelDimethicone-Methacrylatcopolymer, Hydroxypropyldimethiconebehenat, Dimethicone Propyl-PG-Betain, Polyglyceryl 4-lsostearat, Glycerylstearat, Glycerylstearatcitrat, Capryly Dimethicone Ethoxyglucoside.

20. Zubereitung nach einem der vorhergehenden Ansprüche, **dadurch gekennzeichnet, dass** zusätzlich in der Kosmetik übliche Hilfs- und Zusatzstoffe enthalten sind.

21. Zubereitung nach Anspruch 20, **dadurch gekennzeichnet, dass** als Hilfsstoff bzw. Zusatzstoff Konservierungsmittel, Antioxidantien, Mittel zur Regulierung des pH-Wertes, pflanzliche oder tierische Extrakte, Riechstoffgemische, Lichtschutzfilter oder Mischungen der genannten Inhaltsstoffe enthalten sind.

22. Zubereitung nach einem der vorhergehenden Ansprüche, **dadurch gekennzeichnet, dass** das flüchtige Silikon in der Zubereitung in einem Anteil von 10 bis 25 Gew.-% enthalten ist.

23. Zubereitung nach einem der vorhergehenden Ansprüche, **dadurch gekennzeichnet, dass** Pigmente in einem Anteil von 0,1 bis 15 Gew.-% in der Zubereitung enthalten sind.

24. Verfahren zur Herstellung einer Zubereitung, wie in einem der Ansprüche 1 bis 23 definiert, bei dem man Pigmente und Füllstoffe mit dem flüchtigen Silikon vermischt, ggf. Konservierungsmittel und Feuchthaltemittel in Wasser einarbeitet, Silikonphase und wässrige Phase homogenisiert, um eine Emulsion zu bilden, ggf. vor oder nach der Emulgierung Emulgator zufügt und Verdicker und filmbildende Polymere zugibt und anschließend in Behälter abfüllt.

25. Verwendung einer Emulsion mit einer wässrigen Phase, die eine filmbildende Polymerkombination enthält, und einer emulgierten Phase, die ein oder mehrere flüchtige Silikone, mindestens ein Pigment und ggf. Füllstoffe enthält, als faltenkaschierender Überzug auf Haut und/oder Schleimhaut.

26. Verwendung einer Emulsion, wie in einem der Ansprüche 1 bis 23 definiert, als Make-up, Concealer, Foundation, Augencreme oder Lippencreme mit faltenkaschierender Wirkung.

27. Verwendung nach Anspruch 26 um Falten im Gesicht, um die Augen, um die Lippen und/oder Hals zu kaschieren.

## Claims

1. A preparation in the form of an emulsion for application to skin and/or mucous membrane, containing an aqueous phase with a film-forming polymer combination of at least two polymers and an emulsified phase with at least one volatile silicone, at least one pigment and also optionally fillers, wherein the film-forming polymer combination is used in the form of an aqueous solution or dispersion, wherein the film-forming polymer combination has an elastic polymer and a polymer introducing solidity or viscosity, and it is contained, furthermore, in a proportion of 5 to 50 % by weight relative to the total preparation, and wherein at least one globular fine pigment in the form of a light-diffusing pigment is contained as the pigment.

2. A preparation according to claim 1, **characterised in that** the elastic polymer is an acrylate or a derivative thereof, a methacrylate or a derivative thereof, a polyvinyl alcohol and/or a polyvinyl acetate or a copolymer of two or more of the monomers mentioned or a mixture thereof.

3. A preparation according to claim 1 or 2, **characterised in that** the polymer introducing solidity or viscosity is a polyurethane, polyether urethane, polyester urethane, polyvinyl pyrrolidone or a mixture thereof.

4. A preparation according to one of the preceding claims, **characterised in that** the film-forming polymer combination contains a polyurethane acrylate copolymer.

5. A preparation according to one of the preceding claims, **characterised in that** the film-forming polymer combination is a polymer blend of a polyurethane polymer and/or polyvinyl pyrrolidone polymer in combination with an acrylate-, acrylamide-, acrylic ester polymer, a polyvinyl alcohol and/or polyvinyl acetate.

6. A preparation according to claim 1, **characterised in that** the polymer combination is selected from octadecene/MA copolymer/diethylhexyl sebacate, acrylates copolymer, polyurethane acrylates copolymer, lauryl dimethicone, PVP/dimethyl aminoethyl methacrylate/polycarbamyl/polyglycol ester, PVP/dimethiconylacrylate/polycarbamyl/polyglycol ester, PPG/IPDI/DPMA crosspolymer, alkylacrylate copolymer, acrylates/stearyl acrylate/dimethicone methacrylate copolymer, polyvinyl stearyl ether, acrylates/lauryl acrylate/stearyl acrylate/ethylamine oxide methacrylate copolymer, polyurethane and mixtures thereof.

7. A preparation according to one of the preceding claims, **characterised in that** a combination of one or more polyurethanes and one or more acrylates and/or methacrylates in the form of a polymer blend is present as the film-forming polymer combination.

8. A preparation according to one of the preceding claims, **characterised in that** a combination of film-forming polymers selected from polymers, copolymers and/or crosspolymers of polyvinyl pyrrolidone, polyvinyl alcohol, polyvinyl acetate, polyurethane, polyacrylate and polymethacrylate is contained.

9. A preparation according to one of the preceding claims, **characterised in that** the film-forming polymer combination is contained in a proportion of 5 to 25 % by weight relative to the total preparation.

10. A preparation according to one of the preceding claims, **characterised in that** at least one moistening agent is additionally contained in the aqueous phase.

11. A preparation according to claim 5, **characterised in that** 1,2-propylene glycol, dipropylene glycol, tripropylene glycol, glycerine, diglycerine, triglycerine, butylene glycol, hexylene glycol, sugar, sugar alcohol, mannitol, sorbitol, xylitol, glucose, fructose, sucrose, maltose, carbamide, lactic acid in Na-lactate, sodium PCA or a mix thereof is contained as the moistening agent.

12. A preparation according to one of the preceding claims, **characterised in that** one or more pigments selected from organic pigments, inorganic pigments, lakes of organic colourants, metal powder, coated metal powders, plate-like preparations based on silicon dioxide, aluminium oxide or glass or other mixtures are contained in addition to light-diffusing pigments.

13. A cosmetic preparation according to claim 1, **characterised in that** the volatile silicone is dimethicone, hexamethyldisiloxane, cyclomethicone, dodecamethylcyclohexasiloxane, decamethylcyclopentasiloxane, octamethylcyclotetrasiloxane, or a derivative or a mixture thereof, optionally in combination with ethyl perfluoroisobutyl ether or ethyl perfluorobutyl ether.

14. A preparation according to one of the preceding claims, **characterised in that** a filler is contained.

15. A preparation according to one of the preceding claims, **characterised in that** dimethicone/vinyl dimethicone crosspolymer, polyamide, polyethylene, kaolin, talcum, boron nitride, PMMA, methyl methacrylate crosspolymer, silica, alumina, mica, PTFE, lauroyllysine is contained as the filler.

16. A preparation according to one of the preceding claims, **characterised in that** an active substance is additionally contained.

17. A preparation according to one of the preceding claims, **characterised in that** the preparation is wax-, fat- and oil-free.

18. A preparation according to one of the preceding claims, **characterised in that** an emulsifier is additionally contained.

19. A preparation according to one of the preceding claims, **characterised in that** a non-ionogenic emulsifier is contained that is preferably selected from polyglyceryl-3-methyl glucose-distearate, perfluorononyl dimethicone and derivatives thereof, alkyl dimethicone, acrylate/stearyl acrylate/dimethicone methacrylate copolymer, hydroxypropyl dimethicone behenate, dimethicone propyl-PG-betaine, polyglyceryl 4-isostearate, glyceryl stearate, glyceryl stearate citrate, capryl dimethicone ethoxy glucoside.

20. A preparation according to one of the preceding claims, **characterised in that** in cosmetics standard auxiliary and additional materials are additionally contained.

21. A preparation according to claim 20, **characterised in that** preservatives, antioxidants, means to regulate the pH value, vegetable or animal extracts, scent mixes, light-protection filters or mixtures of the ingredients mentioned are contained as the auxiliary material or additional material.

22. A preparation according to one of the preceding claims, **characterised in that** the volatile silicone is contained in the preparation in a proportion of 10 to 25 % by weight.

23. A preparation according to one of the preceding claims, **characterised in that** pigments are contained in the preparation in a proportion of 0.1 to 15 % by weight.

24. Method for producing a preparation, as defined in one of claims 1 to 23, in which pigments and fillers are mixed with the volatile silicone, optionally preservatives and moistening agents are incorporated in water, the silicone phase and aqueous phase are homogenized in order to form an emulsion, optionally emulsifier is added before or after the emulsification, and thickeners and film-forming polymers are added, and subsequently containers are filled therewith.

25. Use of an emulsion with an aqueous phase containing a film-forming polymer combination and an emulsified phase containing one or more volatile silicones, at least one pigment and optionally fillers, as a wrinkle-concealing coating on skin and/or mucous membrane.

26. Use of an emulsion, as defined in one of claims 1 to 23, as make-up, concealer, foundation, eye cream or lip cream with a wrinkle-concealing effect.

27. Use according to claim 26 in order to conceal wrinkles in the face, around the eyes, around the lips and/or neck.

## Revendications

1. Préparation sous la forme d'une émulsion, destinée à une application sur la peau et/ou la muqueuse, contenant une phase aqueuse comportant une combinaison polymère filmogène composée d'au moins deux polymères et une phase émulsionnée présentant au moins une silicone volatile, au moins un pigment ainsi qu'éventuellement des matières de charge, dans laquelle la combinaison polymère filmogène est utilisée sous la forme d'une solution ou d'une dispersion aqueuse, dans laquelle la combinaison polymère filmogène présente un polymère élastique et un polymère conférant une ténacité ou une viscosité, et est en outre présente selon une proportion comprise entre 5 et 50 % en masse, sur la base de la préparation totale, et dans laquelle est présent en tant que pigment au moins un pigment fin en forme de billes sous la forme de pigments diffusants ou LDP.

2. Préparation selon la revendication 1, **caractérisée en ce que** le polymère élastique est un acrylate ou un dérivé de celui-ci, un méthacrylate ou un dérivé de celui-ci, un alcool de polyvinyle et/ou un acétate de polyvinyle, ou un copolymère de deux ou plus des monomères cités, ou un mélange de ceux-ci.

3. Préparation selon la revendication 1 ou 2, **caractérisée en ce que** le polymère conférant une ténacité ou une viscosité est un polyuréthane, un polyétheruréthane, un polyesteruréthane, une polyvinylpyrrolidone, ou un mélange de ceux-ci.

4. Préparation selon l'une des revendications précédentes, **caractérisée en ce que** la combinaison polymère filmogène contient un copolymère de polyuréthane-acrylate.

5. Préparation selon l'une des revendications précédentes, **caractérisée en ce que** la combinaison polymère filmogène est un mélange polymère composé d'un polymère de polyuréthane et/ou d'un polymère de polyvinypyrrolidone en combinaison avec un polymère d'acrylate, d'acrylamide, d'acrylester, un alcool de polyvinyle et/ou un acétate de polyvinyle.

6. Préparation selon la revendication 1, **caractérisée en ce que** la combinaison polymère est sélectionnée parmi un octadécène / copolymère de MA / sébacate de diéthylhexyle, un copolymère d'acrylate, un copolymère de polyuréthane-acrylate, une lauryldiméthicone, un PVP / aminoéthylméthacrylate de diméthyle / polycarbamyle / polyglycolester, un PVP / acrylate de diméthyconyle / polycarbamyle / polyglycolester, un polymère réticulé de PPG / IPDI / DPMA, un copolymère d'acrylate d'alkyle, un copolymère d'acrylate / acrylate de stéaryle / méthacrylate de diméthicone, un polyvinylstéaryléther, un copolymère d'acrylates / acrylate de lauryle / acrylate de stéaryle / méthacrylate d'éthylamine-oxyde, un polyuréthane, et leurs mélanges.

7. Préparation selon l'une des revendications précédentes, **caractérisée en ce qu'**est présente en tant que combinaison polymère filmogène une combinaison d'un ou de plusieurs polyuréthanes et d'un ou de plusieurs acrylates et/ou méthacrylates sous la forme d'un mélange polymère.

8. Préparation selon l'une des revendications précédentes, **caractérisée en ce qu'**une combinaison de polymères filmogènes est sélectionnée parmi des polymères, copolymères et/ou polymères réticulés de polyvinylpyrrolidone, alcool de polyvinyle, acétate de polyvinyle, polyuréthane, polyacrylate et polyméthacrylate.

9. Préparation selon l'une des revendications précédentes, **caractérisée en ce que** la combinaison polymère filmogène est présente selon une proportion comprise entre 5 et 25 % en masse, sur la base de la préparation totale.

10. Préparation selon l'une des revendications précédentes, **caractérisée en ce que** la phase aqueuse contient en outre au moins un agent humectant.

11. Préparation selon la revendication 5, **caractérisée en ce qu'**est présent en tant qu'agent humectant du 1,2-propylèneglycol, du dipropylèneglycol, du tripropylèneglycol, de la glycérine, de la diglycérine, de la triglycérine, du butylèneglycol, de l'hexylèneglycol, un sucre, un sucre-alcool, du mannitol, du sorbitol, du xylitol, du glucose, du fructose, du saccharose, du maltose, de l'urée, de l'acide lactique dans un lactate de Na, un PCA de sodium, ou un mélange de ceux-ci.

12. Préparation selon l'une des revendications précédentes, **caractérisée en ce qu'**en plus du LDP sont présents un ou plusieurs pigments sélectionnés parmi des pigments organiques, des pigments inorganiques, des formations de pigments à base de colorants organiques, de la poudre métallique, des poudres métalliques enrobées, des préparations en paillettes à base de dioxyde de silicium, d'oxyde d'aluminium, ou du verre ou d'autres mélanges.

13. Préparation cosmétique selon la revendication 1, **caractérisée en ce que** la silicone volatile est une diméthicone, un hexaméthyldisiloxane, une cyclométhicone, un dodécaméthylcyclohexasiloxane, un décaméthylcyclopentasiloxane, un octaméthylcyclotétrasiloxane, ou un dérivé ou un mélange de ceux-ci, éventuellement en combinaison avec un perfluoroisobutyléther d'éthyle ou un perfluorobutyléther d'éthyle.

14. Préparation selon l'une des revendications précédentes, **caractérisée en ce qu'**est présente une matière de charge.

15. Préparation selon l'une des revendications précédentes, **caractérisée en ce qu'**elle contient en tant que matière de charge un polymère réticulé de diméthicone/vinyldiméthicone, un polyamide, un polyéthylène, du kaolin, du talc, du nitrure de bore, du PMMA, un polymère réticulé de méthacrylate de méthyle, de la silice, de l'alumine, du mica, du PTFE, de la lauroyllysine.

16. Préparation selon l'une des revendications précédentes, **caractérisée en ce qu'**elle contient en outre un principe actif.

17. Préparation selon l'une des revendications précédentes, **caractérisée en ce que** la préparation est exempte de cire, de graisse et d'huile.

18. Préparation selon l'une des revendications précédentes, **caractérisée en ce qu'**elle contient en outre un émulsifiant.

19. Préparation selon l'une des revendications précédentes, **caractérisée en ce qu'**elle contient un émulsifiant non ionogène qui est de préférence sélectionné parmi le polyglycéryl-3-méthylglucose-distéarate, la perfluorononyldiméthicone et ses dérivés, une alkyldiméthicone, un copolymère d'acrylate/acrylate de stéaryle/méthacrylate de diméthicone, du hydroxypropyldiméthiconebéhénate, de la propyl-PG-bétaïne de diméthicone, du 4-isostéarate de polyglycéryle, du stéarate de glycéryle, du stéarate-citrate de glycéryle, un éthoxyglucoside de capryl-diméthicone.

20. Préparation selon l'une des revendications précédentes, **caractérisée en ce qu'**elle contient en outre des excipients et additifs usuels en cosmétique.

21. Préparation selon la revendication 20, **caractérisée en ce qu'**elle contient en tant qu'excipient ou additif des conservateurs, des antioxydants, des agents de régulation de la valeur du pH, des extraits végétaux ou animaux, des mélanges de substances odorantes, des filtres de protection solaire ou des mélanges des ingrédients cités.

22. Préparation selon l'une des revendications précédentes, **caractérisée en ce que** la silicone volatile est présente dans la préparation selon une proportion comprise entre 10 et 25 % en masse.

23. Préparation selon l'une des revendications précédentes, **caractérisée en ce que** des pigments sont présents dans la préparation selon une proportion comprise entre 0,1 et 15 % en masse.

24. Procédé de fabrication d'une préparation telle que définie dans l'une des revendications 1 à 23, dans lequel on mélange des pigments et des matières de charge avec la silicone volatile, on incorpore éventuellement dans de l'eau des conservateurs et des agents humectants, on homogénéise la phase de silicone et la phase aqueuse afin de former une émulsion, on ajoute éventuellement avant ou après l'émulsionnement un émulsifiant et on ajoute un épaississant et des polymères filmogènes, puis on conditionne ensuite dans des récipients.

25. Utilisation d'une émulsion comportant une phase aqueuse qui contient une combinaison polymère filmogène et une phase émulsionnée qui contient une ou plusieurs silicones volatiles, au moins un pigment et éventuellement des matières de charge en tant que produit destiné à masquer les rides sur la peau et/ou la muqueuse.

26. Utilisation d'une émulsion telle que définie dans les revendications 1 à 23 en tant que maquillage, cache-cernes, fond de teint, crème pour les yeux ou crème pour les lèvres présentant un effet de masquage des rides.

27. Utilisation selon la revendication 26 pour masquer les rides du visage, du contour des yeux, du contour des lèvres et/ou du cou.
